# EUROPEAN PATENT APPLICATION

(11) **EP 2 821 043 A1**
(43) Date of publication of application: **07.01.2015**
(21) Application number: 13174693.5
(22) Date of filing: 02.07.2013
(51) Int. Cl.: A61F 13/511, A61F 13/514, D04H 1/54, D04H 3/14, D06C 15/02

(54) **Non-woven fabric and process for forming the same**

(71) Applicant: Fitesa Germany GmbH, 31224 Peine (DE)
(72) Inventor: Novarino, Elena, 30539 Hannover (DE); Siebner, Harald, 38122 Braunschweig (DE); Hartl, Helmut, 38124 Braunschweig (DE); Teschner, Florian, 30171 Hannover (DE)
(74) Representative: DeltaPatents B.V.

(57) **Abstract**

The present invention relates to a non-woven fabric comprising a first non-woven web suitable for use in an absorbent article, which web comprises a side which is provided with a first pattern of individualised bonded areas which defines a second pattern of non-bonded areas which non-bonded areas have a hexagonal type of shape, and wherein the surface of the bonded areas is in the range of 10-30 % of the total surface of the side and the surface of the non-bonded area is in the range of from 70-90 % of the total surface of the side. The present invention further relates to an absorbent article comprising the present non-woven fabric, and a process for forming the non-woven fabric.

## Description

### FIELD OF THE INVENTION

The present invention relates to a non-woven fabric, an absorbent article comprising the non-woven fabric, and a process for forming the non-woven fabric.

### BACKGROUND OF THE INVENTION

Non-woven fabrics are widely applied in disposable absorbent articles for personal care or hygiene. In such articles the appearance of softness is of great importance since it reassures the wearer or caregiver that the article will be experienced as comfortable.

In WO 2012/024576 A1, an absorbent article adapted to be worn about a wearer's lower torso is described which aims to enhance the perceived softness of the absorbent article. The absorbent article described in said document comprises a liquid permeable top sheet, a liquid impermeable back sheet and an absorbent core disposed between the top sheet and the back sheet. The liquid impermeable back sheet comprises a laminate of a wearer-facing layer of liquid impermeable, vapour permeable polymeric film and a garment-facing layer of a non-woven web. The non-woven web is being impressed with a first pattern of bond impressions in the shape of diamonds, which first pattern defines a second pattern of unbounded raised regions which also have the shape of diamonds. In this respect reference is made to Figures 3A-4B. In the process for manufacturing the non-woven web a hydroentangling or hydroengorgement process is required to increase loft and/or calliper, enhancing visual and tactile softness signals. A drawback of such hydroentangling or hydroengorgement process is, however, that it adds considerably to the manufacturing costs of the absorbent articles. Moreover, the softness of said absorbent articles leaves room for improvement.

WO 2006/048173 describes a loop-forming nonwoven material for a mechanical closure system. The fabrics are thermally bonded with a first pattern of bond impressions that create a second pattern of larger unbonded raised regions and a third pattern of smaller unbonded areas. The impressions are a combination of trilobally and linearly shaped geometry. This provides a positive impact on the mechanical stability of the fabric but has the disadvantage that it limits the drapability, an important feature for softness perceiveness.

Object of the present invention is to provide a non-woven fabric suitable for use in an absorbent article having a simplified pattern of bonded and non-bonded areas which can more easily be manufactured, and which at the same time shows an improved softness.

It is a further object of the present invention to provide a dimensionally stable non-woven fabric with improved softness and a high tensile strength.

Another object of the present invention is to provide a non-woven fabric that has enough shear strength to be used as textile backsheet or landing zone.

It is yet another object of the present invention to non-woven fabrics that can be used as wet and dry wipes.

### SUMMARY OF THE INVENTION

It has now been found that this can be established when use is made of a particular pattern of bonded and non-bonded areas.

Accordingly, the present invention relates to a non-woven fabric comprising a non-woven web suitable for use in an absorbent article, which web comprises a side which is provided with a first pattern of individualised bonded areas which defines a second pattern of non-bonded areas which non-bonded areas have a hexagonal type of shape, and wherein the surface of the bonded areas is in the range of 10-30 % of the total surface of the side and the surface of the non-bonded area is in the range of from 70-90 % of the total surface of the side.

A major advantage of the present invention resides in the fact that the present pattern is relatively simple to make and it has an improved perceived softness. Moreover, the three-dimensional surface of the non-woven fabric of the present invention provides an aesthetically pleasing appearance for its user. An additional major advantage is the fact that present non-woven fabrics display an improved softness and at the same time a high tensile strength. This is surprising since it is generally acknowledged that softness and dimensional stability (i.e. high tensile strength) of a thermobonded nonwoven fabric are features that mutually exclude each other.

### DETAILED DESCRIPTION OF THE INVENTION

In accordance with the present invention the non-woven fabric comprising a non-woven web which comprises a side which is provided with a first pattern of individualised bonded areas which defines a second pattern of non-bonded areas which non-bonded areas have a hexagonal type of shape, and wherein the surface of the bonded areas is in the range of 10-30 % of the total surface of the side and the surface of the non-bonded area is in the range of from 70-90 % of the total surface of the side. The high surface of the non-bonded areas to be used according to the present invention provides the attractive high softness. Moreover, the large unbonded areas allow for the fiber to bulk up and increase the bulkiness of the fabric. This is perceived as an even higher softness from both visual and the tactile perspective. Preferably, the surface of the non-bonded area is in the range of from 75-90 % of the total surface area of the side. Preferably, the surface of the non-bonded area is more than 75% but not more than 90% of the total surface area of the side. More preferably, the surface of the non-bonded area is in the range of from 80-90 % of the total surface area of the side.

The surface of the bonded area is preferably in the range of from 10-25 % of the total surface area of the bonding pattern, more preferably in the range of from 10-20 % of the total surface area of the side.

The first pattern of individualised bonded areas defines the second pattern of non-bonded areas which non-bonded areas have a hexagonal type of shape.

Preferably, the side of the non-woven fabric is only provided with the first pattern and the second patterns, meaning that no further pattern of bonded or non-bonded areas is provided on the side of the non-woven fabric.

Therefore, the present invention also relates to a non-woven fabric comprising a non-woven web suitable for use in an absorbent article, which web comprises a side which is provided with an overall pattern which consists of a first pattern and a second pattern, in which the first pattern is a pattern of individualised bonded areas which defines the second pattern of non-bonded areas which non-bonded areas have a hexagonal type of shape, and wherein the surface of the bonded areas is in the range of 10-30 % of the total surface of the side and the surface of the non-bonded area is in the range of from 70-90 % of the total surface of the side.

Preferably, the individualised bonded areas have a non-linear shape. In the context of the present application a non-linear shape is defined as a shape which is not linear as such or does not contain one or more linear parts.

Suitably, the non-bonded areas have the shape of a regular or an irregular hexagon in which one or more sides have a different length. Preferably, the non-bonded areas have a regular hexagonal shape.

Suitably, the non-bonded areas have a surface in the range of from 20-50 mm2, preferably in the range of from 22-45 mm2, and more preferably in the range of from 23-40 mm2.

Suitably, the non-woven web has a basis weight in the range of from 5-80 g/m2, preferably in the range of from 6-50 g/m2, and more preferably in the range of from 8-30 g/m2.

The individualised bonded areas suitably have a symmetrical shape such as a circle, diamond, rectangle, square, oval, triangle, heart, moon star, hexagonal, octagonal or another polygon shape. Preferably, the bonded areas have a circle or hexagonal shape. More preferably, the bonded areas have a circle shape.

The bonded areas suitably have a maximum width in the range of from 0.7-1.5 mm, preferably in the range of from 0.75-1.25 mm, and more preferably in the range of from 0.8-1.2 mm.

Suitably, the bonded areas have a surface in the range of from 0.38-1.77 mm2, preferably in the range of from 0.44-1.22 mm2, and more preferably in the range of from 0.50-1.13 mm2. The discrete non-bonded areas suitably have a depth in the range of from 0.4-1.5 mm, preferably in the range of from 0.4-0.9 mm, more preferably in the range of from 0.4-0.8 mm, and most preferably in the range of from 0.5-0.7 mm.

Suitably, an even number of bonded areas defines an individual non-bonded area. Preferably, the individual non-bonded areas are defined by 6, 12, 18 or 24 individualised bonded areas, more preferably 12, 18 or 24 individualised bonded areas, and most preferably by 12 individualised bonded areas.

Suitable non-woven webs may be produced by any of the means known to the art for making a non-woven.

The non-woven web may be a single layer or multi-layer non-woven having, for example, at least one layer of a spunbonded web joined to at least one layer of a meltblown web, a carded web, or other suitable material. Suitably, the non-woven fabric according to the present invention comprises in addition a second non-woven web.

The non-woven webs may be extensible, elastic, or non-elastic. The non-woven webs may be spunbonded webs, meltblown webs, air-laid webs, or carded webs. If the non-woven web is a web of meltblown fibers, it may include meltblown microfibers.

The non-woven webs particularly suitable are made from fibers of thermoplastic polymers such as polyolefins, polyesters, ethylene copolymers, propylene copolymers, butene copolymers, and combinations thereof, but may also comprise natural fibers such as wood, cotton, or rayon in combination with thermoplastic fibers. The nonwoven web may also be a composite made up of a mixture of two or more different fibers or a mixture of fibers and particles.

The fibers are suitably joined by bonding to form a coherent web structure. Suitable bonding techniques include, but are not limited to, chemical bonding and thermal bonding, for example thermal calendering or bonding by a hot gas stream.

A wide range of suitable polyolefins can be used in the present invention. Suitable examples include polyethylene, polypropylene, polybutadiene, poly(ethylene-butene), poly(ethylene-hexene), poly(ethylene-octene), poly(ethylene-propylene) block polymers such as styrene/isoprene/styrene and styrene/polybutadiene/styrene. The polyolefins may comprise a homopolymer or a copolymer such as propylene-α-olefins copolymers. In particularly, the latter copolymers can attractively be used in the present invention. Preferred are polyolefin materials that comprise a propylene-α-olefin copolymer and a propylene homopolymer.

The melt flow rate (MFR) of the polyolefin material is suitably less than 90 dg/min. The MFR is determined using ASTM test method D1238, 2.16 kg. Preferably, the MFR of the polyolefin material is in the range of from 15-50 dg/min, more preferably in the range of from 15-35 dg/min.

Preferably, the fibers are formed from polyethylene, polypropylene, a co-polymer of polyethylene and polypropylene, a blend of polyethylene and polypropylene, a polyester, co-polymers of polyesters and/or blends of polyesters.

Suitably use is made of a propylene-based or ethylene-based homopolymer or a copolymer. In the case of propylene-based polymers the polymers may comprise comonomer-derived units selected from ethylene and C4-C10 α-olefins. In the case of ethylene-based polymers the polymers may comprise comonomer-derived units selected from C3-C10 α-olefins. Suitable examples of polyolefin materials include propylene homopolymers, ethylene homopolymers, propylene copolymers and ethylene copolymers such as linear low density polyethylene (LLDPE), high density polyethylene (HDPE), and low density polyethylene (LDPE).

Suitable polyesters can be aliphatic polyesters such as, e.g. polylactic acid, or aromatic polyesters such as polyethylene terephtalate (PET) and poly(trimethylene terephtalate (PTT).

Besides additives already contained in the employed polymers, addition of further additives is possible to provide additional properties to the fibers. Suitable further additives include thermal stabilizers, light stabilizers, slip additives, waxes, and additives to make the fabrics either hydrophilic or hydrophobic. The addition of filler materials can sometimes also be of advantage. Suitable filler materials include organic and inorganic filler materials. Suitable examples of inorganic filler materials include minerals such as calcium carbonate, metals such as aluminium and stainless steel. Suitable examples of organic filler materials include sugar-based polymers.

Various fiber cross-sections are possible. As a rule, round fiber cross-section is preferred, but also tri- and multilobal shaped fibers can advantageously be used. Other suitable fiber cross-sections include triangular, bone-shaped, moon-shaped, hollow-fibers, and ribbon-shaped cross-sections.

The fibers from which the non-woven webs are made can suitable be single component or multicomponent fibers such as bicomponent fibers. Suitable examples of multicomponent fibers include symmetric and eccentric core/sheath fibers, side-by-side fibers of A/B or A/B/A-structure, segmented pie fibers, island-in-a-sea fibers, and striped fibers. Preferred are bicomponent fibers where the two components are arranged in a symmetric core-sheath way or in a side-by-side way. Most preferred are core-sheath bicomponent fibers comprising polyethylene and polypropylene, but any other combination of other suitable polymers is possible as well, for example combinations of polyesters with polyolefins. In general, for core-sheath fibers, the core comprises the component with the higher melting point and the sheath comprises the component with the lower melting point, but it can be advantageous to have it in the reversed way. In a preferred embodiment, the bicomponent fiber has a core of polypropylene and a sheath of polyethylene. In preferred embodiments, the bicomponent fiber comprises from 10% to 90% by weight of the higher melting component in the core and from 90% to 10% by weight of the lower melting component in the sheath. Most preferably, the bicomponent fiber has from 30% to 70% by weight of the higher melting component in the core. The bicomponent fibers may contain different types of polypropylene. More preferably, the bicomponent fiber has a core of a polypropylene which has a higher melting point and a sheath of a polypropylene which has a lower melting point. In another preferred embodiment, a side-by-side-bicomponent fiber comprises two polypropylenes that differ in melt temperature or melt flow.

The fibers can be made according to spinning technologies known in the art. Most conveniently employed are spunbond and meltblown processes, from which the non-woven fabrics can directly be formed.

Spunbond fibers are generally produced by extruding a molten polymer through a large spinneret having several thousand holes per linear meter or from banks of smaller spinnerets, for example, containing as few as 40 holes. After exiting the spinneret, the molten fibers are quenched by a cross-flow air quench system, then pulled away from the spinneret and attenuated by high speed air. Lay-down of the filaments to create a nonwoven layer occurs on a permeable transport belt. Spunbond fibers are generally continuous and range in fiber diameter between ca. 10-100 µm.

Meltblown fibers on the other hand are generally much smaller in diameter and usually range between 0.5-10 µm. Additionally, meltblown fibers are considered to be mainly discontinuous.

A meltblowing process is a process in which fibers are formed by extruding a molten thermoplastic material through a plurality of fine, usually circular, die capillaries as molten threads or filaments into a high velocity, usually heated, gas streams which attenuate the filaments of molten thermoplastic material to reduce their diameter. The meltblown process normally has the filaments in single row of filaments across the width of the die. Thereafter, the meltblown fibers are carried by the high velocity gas stream and are deposited on a collecting surface to form a web of randomly dispersed meltblown fibers. Meltblown fibers are microfibers which may be continuous or discontinuous.

The non-woven fabric in accordance with the invention can additionally be treated to add specific properties. Most common are topical treatments to make the fabric either hydrophilic or to make it hydrophobic. Most common is the treatment of the fabric with either hydrophilic surfactants or with a fluorocarbon or a silicon material. In the context of the present invention a surface of a non-woven fabric or non-woven web is "hydrophilic" when the contact angle of water disposed on that surface is less than about 90 and a surface is "hydrophobic" when the contact angle of water disposed on that surface is greater than or equal to 90.

The non-woven fabric according to the invention can consist of only one type of fibers or fiber layers, e.g. a spunbond layer, but it suitably can comprise additional non-woven layers which may differ from each other. Suitable multi-layer fabrics may include one or more spunbond layers (S) and meltblown layers (M), such as SMS, SMMS, SSMMS, etc. adhered to a nonwoven fabric according to the present invention. Usually, these multilayer fabrics are made in one step on a single line with multiple beams, which generally encompass a combination of spunbond and meltblown beams. In some cases it might be advantageous or technically necessary to make a multiple layer according to the invention in two or more separate steps.

The use of spunbond layers that differ in their fiber cross-section or in their fiber type are possible. Thus, it is also possible to combine a layer of trilobal filaments with a layer of round fibers, or to combine a core-sheath bicomponent layer with a side-by-side biocomponent layer.

The non-woven fabric according to the present invention and that might consist of one or more single layers can suitably have a basis weight in the range between 5-80 grams/m2, preferably in the range of from 6-50 grams/m2.

The present invention also relates to an absorbent article comprising the non-woven fabric according to the present invention.

Suitably, the absorbent article according to the present invention is a disposable absorbent article selected from the group consisting of hygiene articles, incontinence articles, diapers, wipes and fem-care articles. Suitable disposable absorbent articles according to the present invention include those selected from the group consisting of baby diapers, pull-ups, training pants, hygiene closure systems, adult incontinence briefs and diapers, panty liners, sanitary napkins, medical garments, and bandages. Suitable wipes can comprise wet and dry wipes for hygiene and home care, cleaning wipes, industrial wipes, oil absorbing wipes, and the like.

Disposable absorbent articles are absorbent articles which are not intended to be laundered or otherwise restored or reused as absorbent articles. Generally, such absorbent articles comprise a back sheet, a top sheet and an absorbent core which is arranged between the back sheet and the top sheet. The back sheet will have an integral landing zone or will work as a landing zone on its own, and comprises a nonwoven web which is arranged at the outermost portion of the absorbent article and prevents liquid from passing through the absorbent articles, whereas the exudates from a wearer's lower torso will pass through the top sheet and are absorbed in the absorbent core. An additional function of the top sheet is to provide skin comfort.

Further, the disposable absorbent article will usually comprise a hook or loop fastening system to fasten the disposable absorbent article around the wearer's lower torso. When used as the loop fastening system or hook for a disposable personal care absorbent article, the present non-woven fabric can be bonded or attached to the outer layer or back sheet of the article as a discrete patch of loop material. Alternatively, the present non-woven fabric can form the entire outer layer or back sheet of such a disposable personal care absorbent article and function as a landing zone. The hook or loop fastening system can be any of a wide variety of commercially available hook or loop fastening systems.

The non-woven fabric according to the present invention can suitably be part of a top sheet, back sheet, landing zone and/or a loop fastening member. Preferably, the present non-woven fabric is part of a back sheet and/or a landing zone.

The present invention also relates to a process for forming the present non-woven fabric.

Accordingly, the present invention also relates to a process for forming the non-woven fabric comprising the steps of:
(a) forming a non-woven web; and
(b) feeding the non-woven web into a nip defined between oppositely positioned first and second rolls, whereby at least one of the rolls has a patterned outer surface to apply a bonding pattern to the first non-woven web, whereby the bonding pattern comprises the first and second pattern as defined hereinbefore.

The present invention further relates to a process for forming a non-woven fabric according to the present invention comprising the steps of:
(a) forming a first non-woven web;
(b) forming a second non-woven web;
(c) feeding the first non-woven web and the second non-woven web into a nip defined between oppositely positioned first and second rolls, whereby at least one of the rolls has a patterned outer surface to apply a bonding pattern which comprises the first and second pattern as defined hereinbefore;
   and
(d) bonding the first and second non-woven web together to form the non-woven fabric.

Alternatively, the first and second non-woven web may be pre-bonded before feeding the non-woven fabric so formed within the nip formed by the rolls.

Hence, the present invention also relates to a process for forming a non-woven fabric according to the present invention comprising the steps of:
(a) forming a first non-woven web;
(b) forming a second non-woven web;
(c) bonding the first and second non-woven web together to form a non-woven fabric; and
(d) feeding the non-woven fabric as formed in step (c) into a nip defined between oppositely positioned first and second rolls, whereby at least one of the rolls has a patterned outer surface to apply a bonding pattern to the first non-woven web, whereby the bonding pattern comprises the first and second pattern as defined hereinbefore; and
(e) recovering the non-woven fabric.

The rolls to be used in the processes according to the present invention are suitably right circular cylinders that can be formed of any suitable, durable material. Such rolls will be operated in ways known in the art.

The locations of the oppositely positioned rolls can suitably be varied to form the nip between the rolls. The nip pressure within nip can suitably be varied depending upon the properties of the one or more non-woven webs to be processed. The same is true for the necessary temperature of the calender rolls, which has to be adjusted according to the required final properties and the kind of fibers to be bonded.

The bonded areas are suitably formed by means of melt-fusing by controlling the temperature of at least one of the rolls. The temperature of the outer surface of at least one of the rolls can be adjusted by heating or cooling the rolls. The heating and cooling may affect the features of the web(s) being processed and the degree of bonding of single or multiple webs being passed through the nip formed between the respective rolls.

One of the rolls to be used will contain a bonding pattern on its outermost surface comprising a continuous pattern of land areas defining a plurality of discrete openings, apertures or holes. Each of the openings in the one or more rolls will form a discrete unbonded area in at least one side of the non-woven fabric or non-woven web. The other roll will suitably have an outer surface which is much smoother than the other roll. Preferably, the outer surface of the other roll will be smooth or flat. The rotational speeds of the respective rolls are substantially identical.

Hereafter the invention will be further illustrated by the non-limiting drawing.

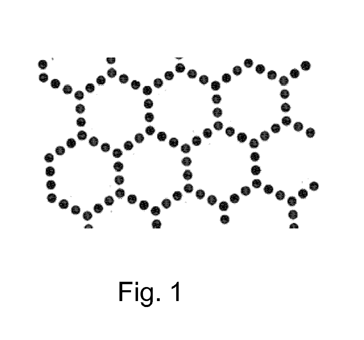

In Figure 1, a side of a non-woven fabric is shown having a bond pattern which comprises a first and second pattern in accordance with the present invention. The first pattern of individualised bonded areas 1 defines a second pattern of non-bonded areas 2. The individualised bonded areas 1 are in the form of circles and the non-bonded areas 2 have a hexagonal type of shape.

## Claims

1. A non-woven fabric comprising a non-woven web suitable for use in an absorbent article, which web comprises a side which is provided with a first pattern of individualised bonded areas which defines a second pattern of non-bonded areas which non-bonded areas have a hexagonal type of shape, and wherein the surface of the bonded areas is in the range of 10-30 % of the total surface of the side and the surface of the non-bonded area is in the range of from 70-90 % of the total surface of the side.

2. A non-woven fabric according to claim 1, wherein the surface of the non-bonded area is in the range of from 75-90 % of the total surface area of the bonding pattern.

3. A non-woven fabric according to claim 2, wherein the surface of the non-bonded area is in the range of from 80-90 % of the total surface area of the bonding pattern.

4. A non-woven fabric according to any one of claims 1-3, wherein the surface of the bonded area is in the range of from 10-25 % of the total surface area of the bonding pattern.

5. A non-woven fabric process according to any one of claims 1-4, wherein the bonded areas have a circle, diamond, rectangle, square, oval, triangle, heart, moon star, hexagonal, octagonal or another polygon shape.

6. A non-woven fabric according to claim 5, wherein the bonded areas have a circle or hexagonal shape.

7. A non-woven fabric according to claim 6, wherein the bonded areas have a circle shape.

8. A non-woven fabric according to any one of claims 1-7, wherein the first non-woven web comprises fibers which are formed from a thermoplastic material selected from the group consisting of polyesters, polyamides, polyolefins, polyurethanes, and any mixture thereof.

9. A non-woven fabric according to claim 8, wherein the fibers are formed from polyethylene, polypropylene, a co-polymer of polyethylene and polypropylene, a blend of polyethylene and polypropylene" a polyester, co-polymers of polyesters and/or blends of polyesters.

10. A non-woven fabric according to claim 8 or 9, wherein the fibers are single component or multi-component fibers.

11. A non-woven fabric according to any one of claims 1-10 comprising additional non-woven webs.

12. An absorbent article comprising the non-woven fabric according to any one of claims 1-11.

13. An absorbent article according to claim 12, wherein the non-woven fabric is a back sheet and/or a landing zone of the absorbent article.

14. An absorbent article according to claim 12 or 13, wherein the absorbent article is a disposable absorbent article selected from the group consisting of hygiene articles, incontinence articles, diapers, wipes and fem- care articles.

15. A process for forming the non-woven fabric comprising the steps of:
(a) forming a non-woven web; and
(b) feeding the non-woven web into a nip defined between oppositely positioned first and second rolls, whereby at least one of the rolls has a patterned outer surface to apply a bonding pattern to the first non-woven web, whereby the bonding pattern comprises the first and second pattern as defined in any one of claims 1-10.

16. A process for forming a non-woven fabric according to claim 11 comprising the steps of:
(a) forming a first non-woven web;
(b) forming a second non-woven web;
(c) feeding the first non-woven web and the second non-woven web into a nip defined between oppositely positioned first and second rolls, whereby at least one of the rolls has a patterned outer surface to apply a bonding pattern to the first non-woven web, whereby the bonding pattern comprises the first and second pattern as defined in any one of claims 1-10;
and
(d) bonding the first and second non-woven web together to form the non-woven fabric.
